(19) Europäisches Patentamt / European Patent Office / Office européen des brevets

(11) **EP 2 856 162 B1**

(12) **EUROPEAN PATENT SPECIFICATION**

(45) Date of publication and mention
of the grant of the patent:
**29.08.2018 Bulletin 2018/35**

(21) Application number: **13730303.8**

(22) Date of filing: **03.06.2013**

(51) Int Cl.:
*B03C 1/28* (2006.01)     *G01N 35/00* (2006.01)
*G01N 33/543* (2006.01)   *B01L 3/00* (2006.01)

(86) International application number:
**PCT/GB2013/051473**

(87) International publication number:
**WO 2013/179069 (05.12.2013 Gazette 2013/49)**

(54) **MICROPLATES WITH ENHANCED IMMOBILISATION CAPABILITIES CONTROLLED BY MAGNETIC FIELD**

MIKROPLATTEN MIT VERBESSERTER IMMOBILISIERUNG DURCH MAGNETFELD

MICROPLAQUES AUX POSSIBILITÉS D'IMMOBILISATION RENFORCÉES COMMANDÉES PAR UN CHAMP MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.06.2012 GB 201209944**

(43) Date of publication of application:
**08.04.2015 Bulletin 2015/15**

(73) Proprietor: **The University of Leicester Leicester (GB)**

(72) Inventors:
• **PILETSKY, Sergey Anatoliyovich Cranfield Bedfordshire MK43 0EE (GB)**
• **PILETSKA, Olena Cranfield Bedfordshire MK43 0EE (GB)**
• **GUERREIRO, Antonio Cranfield Bedfordshire MK43 0HL (GB)**
• **POMA, Alessandro Bedford Bedfordshire MK40 1TH (GB)**
• **KARIM, Khalku Cambridge Cambridgeshire CB4 2ED (GB)**
• **PILETSKY, Stanislav Cranfield Bedfordshire MK43 0EE (GB)**

(74) Representative: **Bailey, Sam Rogerson et al Mewburn Ellis LLP City Tower 40 Basinghall Street London EC2V 5DE (GB)**

(56) References cited:
EP-A2- 0 296 415      WO-A2-03/022421
WO-A2-2009/152092    GB-A- 2 239 947
US-A- 4 146 365       US-A1- 2002 098 121

**Description**

TECHNICAL FIELD

The invention relates generally to devices used for the testing of physical, chemical, biological or biochemical properties, characteristics, or reactions. More particularly, the invention describes microplates with magnetic inserts capable of binding to magnetic (ferro- and paramagnetic) nanoparticles and microparticles and use of such microplates in screening and analysis.

BACKGROUND

Microplates (microtiter plates, or multi-well test plates) are generally used for chemical or biological experiments, such as detection and monitoring of biological or chemical reactions, cell growth, toxicity tests, or combinatorial synthesis. Over the years, many microplate formats which contain cavities, wells, raised pads (USA 7,449,307), microcolumns (USA 7,332,328) or inserts of microarrays (USA 7,219,800) have been developed. However, these plates have a number of drawbacks related to their relatively low surface area which allows only small quantity of reagents (e.g. antibodies) to be immobilised on their surface.

To increase the surface area porous patches were adhered to a flat, rigid, non-porous surface on the bottom of the microplate wells (USA 7,384,779, USA 6,040,171). However the porous materials by their nature might obstruct light pathway required for measuring concentration of analytes in the microplate wells. There is a need therefore for improved microplates which would possess a high surface area while providing unobstructed pathway for light passing through the individual wells. One way to increase the quantity of immobilised material is through the use of magnetic nano- and microparticles with high surface area which could be held in the right position in the microplate wells by applying a magnetic field.

The magnetic (or magnetisable) particles are defined here as these capable of being attracted to a permanent magnet. Small magnetic particles (ferromagnetic or paramagnetic) are well known in the art, as is their use in the separations involving biospecific affinity reactions. For many applications, the surface of paramagnetic particles is coated with a suitable ligand or receptor, such as antibodies, lectins, oligo nucleotides, or synthetic ligands (e.g. molecularly imprinted polymers), which can selectively bind a target substance in a mixture with other substances (see USA 4,230,685, USA 4,554,088, and USA 4,628,037).

There are devices on the market which use magnetic forces to separate or mix magnetic particles with immobilised ligands, manufactured by Dynal, Inc. and PerSeptive Diagnostics. Three examples of such devices which are suitable for separating and for mixing magnetic particles in microplates are described in USA 5,779,907, USA 6,954,128B2 and USA 7,632,405. These devices employ permanent (movable) magnets located externally to a microplate walls and are used mainly for separation. The applications of these devices in diagnostics however are limited since they are not compatible with standard microplate readers.

| Patent | Country | Issued | Title |
|---|---|---|---|
| 7,449,307 | USA | Nov 11, 2008 | Raised surface assay plate |
| 7,384,779 | USA | June 10, 2008 | Porous substrate plates and the use thereof |
| 7,332,328 | USA | Feb 19, 2008 | Microcolumn-platform based array for high-throughput analysis |
| 7,219,800 | USA | May 22, 2007 | Modular array arrangements |
| 6,040,171 | USA | March 21, 2000 | Apparatus for analysing biological samples |
| 7,632,405 | USA | Dec 15, 2009 | Apparatus for processing magnetic particles |
| 5,779,907 | USA | July 14, 1998 | Magnetic microplate separator |
| 6,954,128 B2 | USA | Oct 11, 2005 | High performance hybrid magnetic structure for biotechnology applications |
| 4,230,685 | USA | Oct 28, 1980 | Method of magnetic separation of cells and the like, and microspheres for use therein |
| 4,554,088 | USA | Nov 19, 1985 | Magnetic particles for use in separations |
| 4,628,037 | USA | Dec 9, 1986 | Binding assays employing magnetic particles |

US 2002/0098121 describes an apparatus for automated magnetic separation of materials in laboratory trays by insertion of upstanding magnets into interwell spaces on the underside of a multiwell plate.

WO 2009/152092 describes magnetic microplate assemblies for assaying biological activated magnetic particles from a supernatant. The assemblies including a microplate holder which includes a plurality of magnets for attracting the magnetic particles to the wells.

SUMMARY

Broadly the present invention describes microplates with arrays of wells containing magnetic disks, cylinders or other shaped articles with apertures in the centre for optical interrogation of the contacting solution (henceforth these articles will also be referred to as "inserts"). The present invention relates to a microplate according to claim 1, having a plurality of wells, the plate comprising one or more inserts which are magnets for attracting magnetisable nano- and microparticles, and wherein each of said inserts either:

fits removably into one of the wells; or
is integral with or attached to the bottom or side wall inside the well,

the insert having a hole therethrough along the axis of the well.

Preferably, the substrate microplate device comprises: (1) a substrate having a number of holes (wells) arranged in rows and columns; (2) inserts made of magnetic materials that can be adhered to the well sides and/or bottom, the inserts having apertures in the centre through which solution can be placed into the well and brought into the contact with inserts.

The inserts could be made of metals or alloys, organic (preferably polymeric) or inorganic material (e.g. glass or ceramics) with dispersed ferromagnetic particles.

The invention also teaches methods for fabrication of such microplates and their components using co-sintering, entrapment, in-situ polymerisation etc.

The invention also relates to a magnetic insert for use in a microplate as described herein.

The subject microplates find use in a variety of applications, including clinical and environmental assays, high-throughput screening for genomics, proteomics and pharmaceutical applications, point-of-care in vitro diagnostics, molecular genetic analysis and nucleic acid diagnostics, cell separations, and bioresearch generally and high-throughput screening of materials for separation and catalysis.

The following non-limiting numbered statements also describe preferences relevant to the present invention.

1. Design of microtiter plates comprising: (1) a substrate having a number of holes (wells) arranged in rows and columns; (2) inserts of cylindrical or disklike shape made of magnetic materials that can be adhered to the well sides and/or bottom of the well sides with apertures in the centre through which solution can be placed into the well and brought into the contact with inserts.

2. The inserts in statement 1 are loose fitting or tightly bound to the well surface through physical or chemical attachment by means such as thermal-welding, sonic-welding, infrared-welding, solvent-welding or through the use of an adhesive.

3. The materials used in the fabrication of inserts in statements 1-2 made from metals or substances with paramagnetic or superparamagnetic properties

4. The materials used in the fabrication of inserts in statements 1-2 made from organic, e.g. polymeric material, inorganic material or composites, containing substances with paramagnetic or superparamagnetic properties.

5. The material of inserts in statements 1-3 with functional groups capable of covalent attachment of ligand molecules using chemical reaction such as formation of Shiff's base, disulfide bonds, peptide bonds, S-metal bond, formation of esters, reaction with acetals and thioacetals, etc.

6. Use of microtitre plates with inserts in statements 1-5 for the capturing/immobilisation of magnetic particles with chemical (drugs, peptides, antigens, etc) or biological ligands (antibodies, enzymes, nucleic acids, cells, viruses, etc.).

7. Use of microtitre plates with inserts in statements 1-5 for solid phase synthesis, combinatorial chemistry and high-throughput synthesis.

8. Use of microtitre plates with inserts in statements 1-5 in diagnostic tests such as enzyme immunoassays, clinical and environmental assays, high throughput screening for genomics, proteomics, point-of-care in vitro diagnostics, molecular genetic analysis and nucleic acid diagnostics, and bioresearch generally and high throughput screening of materials for separation and catalysis.

9. Use of microtitre plates with inserts in statements 1-5 as a cell culture support in high-throughput screening (HTS) technologies for the discovery and development of new therapeutic drugs.

## BRIEF DESCRIPTION OF THE FIGURES

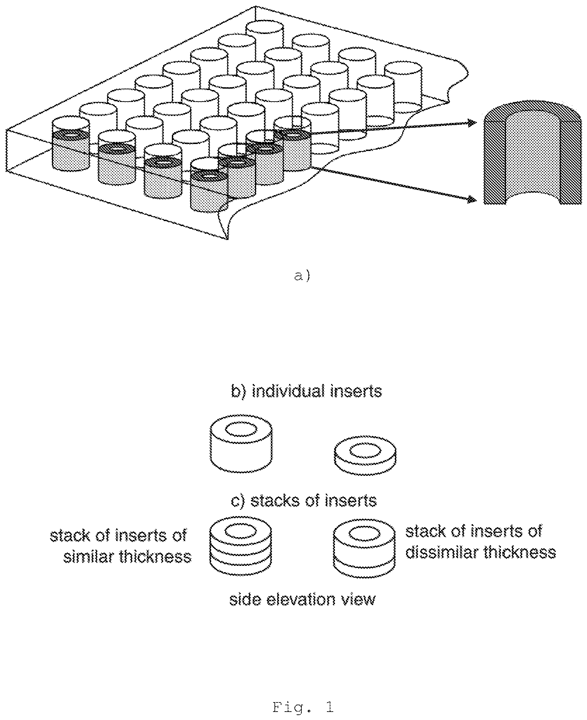

FIG. 1 is a schematic of an embodiment of the invention with part of a generic microplate containing the magnetic inserts inside the wells. On the right is depicted a longitudinal section of the insert showing the central aperture for optical reading. Below (in parts b and c) is a schematic representation of individual inserts of different heights (part b), and of stacks of inserts with similar or dissimilar heights (part c).

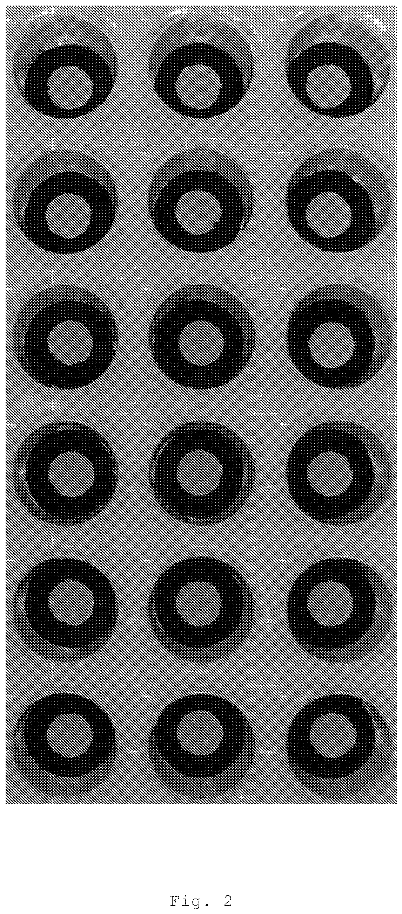

FIG. 2 shows an embodiment of the invention with part of a generic microplate containing an array of the magnetic inserts produced according to an embodiment of the invention as shown in Figure 1 (inserts attached to the inner surface of the microplate wells).

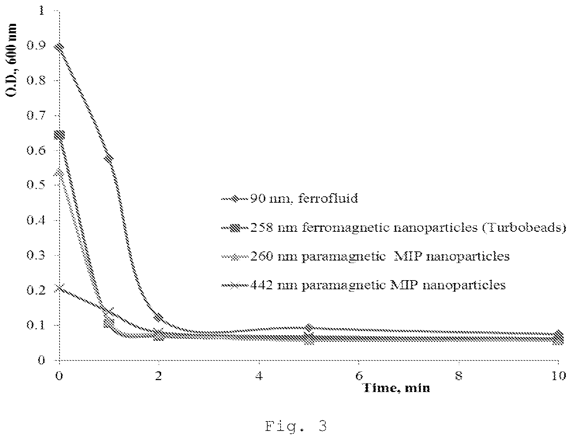

FIG. 3 shows the kinetics of the adsorption of various magnetic materials on the surface of magnetic inserts.

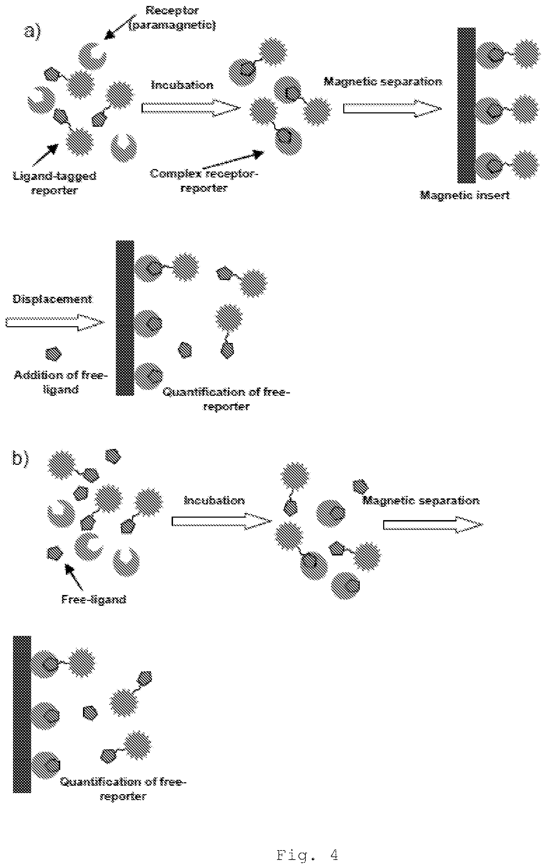

FIG. 4 is a schematic of the colorimetric assay performed in the well of a microplate with magnetic inserts, as produced according to an embodiment of the invention as shown in Fig. 1. The Figure shows the magnetic separation of fluorescent/coloured reporter groups when bound to the paramagnetic material which is coated with receptors (such as antibodies, enzymes, imprinted polymers) or is part of the receptor itself.

## DESCRIPTION OF THE INVENTION

Additional features of the present invention will be revealed in the following detailed description which is merely representative of the invention, and intended to provide an overview for understanding the invention as claimed. Preferred embodiments of the invention will be described with reference to the accompanying drawings.

**The first embodiment** of the present invention describes microplates containing one or more inserts. In preferred aspects, each well of the microplate contains an insert. Microplates or microtiter plates have been in use for a number of years for research and analytical purposes in applications wherein it is required to perform parallel high-throughput chemical and biological assays.

For this purpose biological or chemical molecules (e.g. antibodies) are immobilised on a surface of each well of the microplate. The present invention teaches means for immobilisation of nano- and micro particles with chemical or biological ligands/receptors on the well/insert surface in gradient of magnetic field. According to the present invention, in preferred aspects the substrate plate device comprises: (1) a substrate having a number of holes (wells) arranged in rows and columns; (2) inserts made of magnetic materials that can be adhered to the well sides and/or bottom (Figure 2). Inserts could be loose fitting or be tightly bound to the well surface through physical or chemical attachment by means such as thermal-welding, sonic-welding, infrared-welding, solvent-welding or through the use of an adhesive. The inserts have apertures in the centre through which solution can be placed into the well and brought into the contact with inserts (Figure 1).

The advantages for using inserts inside of the wells (preferred choice in the subject of present invention) are as follows: (i) due to close proximity to the reaction area inserts can be made from relatively weak magnetic materials which would reduce cost of the device; (ii) this arrangement does not interfere with optical analysis (due to the hole through the insert component) which can be performed in standard microplate readers; (iii) the proposed microplates can be used as disposable constructs; (iv) the surface of inserts can be used for performing additional functions, such as e.g. binding to particular ligand or interfering material.

In one aspect of the proposed invention, inserts are blended into the bottom part of the microplate well or attached to the external area of the bottom wall, e.g. as a disc inserted into the bottom of the well or integral with the bottom or walls of the well.

Preferred polymer classes used in the fabrication of substrate holding inserts or blended with inserts are selected from, but not limited to: polyethylene, polystyrenes, polypropylenes, acrylates, methacrylates, polycarbonates, polysulfones, polyesterketones, poly- or cyclic olefins, polychlorotrifluoroethylene, polyesters such as polyethylene terephthalate, chlorine containing polymers such as polyvinylchloride and polyvinylidenechloride, acetal homopolymers and copolymers, cellulose and cellulose nitrate, polyvinylidenefluoride, polytetrafluoroethylene, polyamides, polyimides, polyetheretherketone, polyphenylenesulfide and polyethersulfone, polyurethanes, silicon containing polymers such as polydimethylsiloxane. In addition, the structures can be made from copolymers, blends and/or laminates of the above materials, metal foils, metallised films and metals deposited on the above materials, as well as glass and ceramic materials. In preferred aspects the microplates are made from one of the above listed substrate materials.

A qualitative and quantitative analysis of the composition of the sample fluid placed into the well can be carried out optically through the aperture in the insert. Since the area of the well in the insert aperture is unobstructed for optical reading, the concentration of analyte in solution could be measured using standard microplate readers. The microplates and inserts can be fabricated in situ as a single piece, for example by using injection moulding techniques or thermoforming, or inserts can be manufactured separately and assembled into microplates at later stage.

**The second embodiment** of the present invention describes inserts used in the design and fabrication of microplates. The inserts are preferably disk or cylindrically shaped with an external diameter smaller or equal to the internal diameter of the microplate wells. The internal diameter of the aperture in the centre of the insert should be sufficiently broad so as not to obstruct the light beam of the optical reader. The shapes of the insert and aperture could be circular, elliptical, square, rectangular, polygonal or otherwise as long as it is practical for manufacturing of these devices and suitable for analytical measurement. The inserts could also be prepared in the shape of strips or disks attached to the walls, e.g. to the internal wall of the microplate well.

Preferably, the thickness of the wall of the inserts, i.e. the radial thickness, is 1-5 mm.

As one aspect of the present invention, combinations (stacks) of several disks/cylinders, made of the same or different materials can be used in the same device, e.g. a plurality of disks in the same well. In some aspects, these combinations of disks may differ in properties such as construction material, magnetic properties, or axial thickness. This provides the ability to tailor the properties of the insert at different depths in the well, e.g. to establish different magnetic properties at different depths in the well or to incorporate inserts having different surface functional groups into the same well.

The inserts are designed for the purpose of attracting magnetisable nano- and microparticles. They are fabricated from magnetic material such as iron, cobalt, samarium, neodymium, low-carbon steel or vanadium alloys or rare earth alloys (neodymium-iron-boron or samarium-cobalt). The magnetic materials in the inserts are preferentially dispersed into, or blended with, inorganic or polymer materials, or a combination of inorganic and organic materials. The non-magnetic material used in combination with magnetic material in inserts can be made from a polymer, glass, ceramic material, or combination of these materials. The magnetic inserts could be in part made of the same or different materials as those of the substrate.

The materials used in the fabrication of inserts can be non-porous or could have a porous or gel-like structure. The polymer material may contain vinyl, allyl, styrene, acrylic, methacrylic or acetylene derivatives, with non-exclusive examples of divinylbenzene, divinylnaphthalene, vinylpyridine, hydroxyalkylene methacrylates, ethylene glycol dimethacrylate, vinyl esters of carboxylic acids, divinyl ether, pentaerythritol di-, tri-, or tetramethacrylate or acrylate, trimethylopropane trimethacrylate or triacrylate, alkylene bis acrylamides or methacrylamides, methacrylic and acrylic acid, acrylamide, hydroxyethyl methacrylate, and their mixture, epoxy and urethane resins, molecularly imprinted polymers, chitosan, carbohydrates, oligo- and polysaccharides, peptides, proteins and nucleic acid derivatives, agarose, lipids etc. The gel-like materials could be made of silica gel, glass, polymer, molecularly imprinted polymer, agarose, acrylamide gel, polysaccharides etc.

In one aspect of the proposed invention inserts are fabricated by co-sintering of magnetic and inorganic or polymer particles or by suspension of magnetic particles into the polymer during or prior casting inserts. In one aspect of the proposed invention disk-shape inserts are cut from the magnetic-polymeric film using mechanical tool or laser.

As one aspect of the invention, the surface of the inserts can be activated with functional groups capable of covalent attachment of ligand molecules using chemical reaction such as formation of Schiff's base, disulfide bonds, peptide bonds, S-metal bond, formation of esters, reaction with acetals and thioacetals, etc. For example, using poly(styrene-co-maleic anhydride) (SMA) produces a surface containing a reactive anhydride group to which molecules containing primary amino or hydroxyl groups can be attached by covalent means.

The surface could also have photoreactive groups such as aryl ketones, dithiocarbamates etc. The covalent attachment could also be made through a cleavable unit, which is useful for solid phase synthesis or for recovery of immobilised molecules.

In one aspect of the invention inserts contain biological material such as cells, tissue, bacteria, viruses or their components.

Suitable spacers (e.g. carbon or polyethylene glycol chains) could be used to improve the accessibility of the ligand. In one aspect of the proposed invention inserts contain reagents which can be released when exposed to the solvent including dyes, enzymes, or conjugates, catalysts, substrates, buffer components, surfactants etc. The inserts might have ion-exchanging, adsorbing, catalytic, molecule- or cell- trapping, or cell growing functionalities.

**The third embodiment** of the present invention described magnetic nano- and microparticles used in microplates with magnetic inserts. The magnetic particles preferred for the practice of the invention are nano- and microparticles with paramagnetic or superparamagnetic properties. These particles are attracted to the permanent magnet but do not retain a magnetic field themselves upon removal of the external gradient field and therefore are not attracted to each other. This mechanism allows the particles to disperse in solution in the absence of a magnetic field.

Other particles with ferromagnetic properties also can be used in a combination with microplates with magnetic inserts, however only in the applications where spontaneous aggregation of the magnetic particles is not an issue. The non-exclusive example of such application could be use of magnetic particles for the immobilisation of ligand attached to these particles onto the surface of the inserts. The magnetic particles useful for the practice of present invention are made of inorganic or polymeric material containing a small amount of metal (iron, cobalt, nickel etc.), iron-based oxides, e.g., magnetite, transition metals, or rare earth elements, which causes them to be captured by a magnetic field.

The particles useful for practicing this invention should possess an adequate binding surface capacity for the adsorption or covalent coupling of a specific ligand or receptor.

The preferred diameter of a particle is typically in the range between 0.01-200 $\mu$m. The particular particles useful for this invention are spherical and of uniform size between about 10-500 nm in diameter, and containing magnetic material either specifically confined in their core or uniformly distributed through the entire composition.

**The fourth embodiment** of the present invention describes the application of microplates containing inserts. The microplates with magnetic inserts described herein are used in analytical and biotechnology applications involving holding, concentration, manipulation or separation of magnetic nano- and micro-particles. In one example of proposed application magnetic particles with immobilised ligand (e.g. antibody, protein A, enzyme etc.) are added to the microplate wells where they are captured by the magnetic insert.

The quantity of ligand immobilised on the insert surface can be easily controlled by the quantity of particles added to the wells. Another example of a proposed application of microplates with magnetic inserts involves removal of the interfering matrix (which has e.g. strong optical absorbance or fluorescence), captured by magnetic particles with appropriate affinity ligands from the detection area in the centre of the microplate wells. Yet another example of an intended application involves performing an assay when the free analyte is competing in binding between ligands immobilised onto coloured (or fluorescent) beads and magnetic particles.

The concentration of the analyte then can be measured by measuring residual optical absorbance or fluorescence (originated from non-bound or displaced coloured or fluorescent reporter) in the microplate well (see Figure 4). The examples of target analytes are: cells, cell components, cell subpopulations (both eukaryotic and prokaryotic), bacteria, viruses, parasites, antigens, proteins, including antibodies, nucleic acid sequences etc. Many more applications of the microplates with magnetic inserts for holding, separation of magnetic nano- and micro particles as well as assays which explore magnetic particles are known to the practitioners of the art.

The microplates with inserts may be employed for the immobilisation of ligands (antibodies, enzymes, etc.; antigens; drugs, etc.), in diagnostic tests such as enzyme immunoassays, in affinity chromatography, as an enzyme catalyst in biotechnology, or as a cell culture support, in clinical and environmental assays, high throughput screening for genomics, proteomics, point-of-care in vitro diagnostics, molecular genetic analysis and nucleic acid diagnostics, and bioresearch generally and high throughput screening of materials for separation and catalysis.

In one aspect of the invention the said microplates are used in high-throughput screening (HTS) technologies for the discovery and development of new therapeutic drugs. Other possible applications for the said microplates are in combinatorial chemistry, solid-phase and high-throughput synthesis. Methods of detection using said microplates include, but are not intended to be limited to, changes in colour, light absorption, or light transmission, pH, conductivity, fluorescence, change in physical phase or the like. The apparatus and method of the present invention may be used in bioassays including immunoassays and nucleic acid probe assays.

In the figures, Fig. 1 shows a schematic view of a microplate with cylindrical magnetic inserts in some of the wells. An enlarged and cut-away version of one of the inserts is also shown which illustrates the annular nature of the insert allowing liquid to be inserted into the central "core" of the insert component and allowing an pathway for optical interrogation of the liquid in this region using standard optical detection apparatus.

Part b) of Fig. 1 shows inserts of different axial thicknesses which can be useful for wells of different depths or as components in a stack of inserts such as those shown in part c) of Fig. 1. As noted above, stacks of inserts can be used to vary the properties of the well along its axial length, e.g. to vary the magnetic properties or to provide differing surface activities of the inserts along the axial length of the well.

Fig. 2 shows a microplate with magnetic disks inserted into each well and adhered to the internal bottom surface of the well.

Fig. 4 shows schematics of the proposed colorimetric assay which involves microplates with magnetic inserts and reporter fluorescent/coloured nanoparticles with immobilised ligand. The receptors, which can be of natural origin (i.e. antibodies, enzymes), or synthetic, such as molecularly imprinted polymers (MIPs), are attached to, or include paramagnetic functionalities in order to aid in the magnetic separation step. Figure 3a describes the assay performed in displacement format and Figure 3b describes the assay performed in competition format.

The options and preferences described herein in terms of one embodiment or aspect may be freely combined, insofar as they are compatible, with options and preferences from other embodiments and aspects.

While advantageous embodiments have been chosen to illustrate the invention, it will be understood by those skilled in the art that various changes and modifications can be made therein without departing from the scope of the invention as defined in the corresponding embodiments. The present invention will now be further particularly described with reference to the following, non-limiting examples.

EXAMPLES

**Example 1** - **Preparation of disposable magnetic microtiter plates.**

The disks with external diameter- 6.5 mm, internal diameter- 3 mm were cut from flexible magnetic sheets with self-adhesive backing (0.5 mm thick) (Polarity Magnets, UK) using an infrared laser. The dimension of the disks was selected in order to fit into the wells of the standard microtiter plate with flat bottom.
The magnetic inserts were fixed on the bottom of the wells using adhesive side (Figure 2). After the modification with magnetic inserts the microtiter plates were ready for the testing which involves the magnetic beads.

**Example 2** - **Performance of the magnetic particles with different coating in microplate with magnetic inserts.**

100 $\mu$l of the aqueous suspension of magnetic nanomaterials in 1 mg/ml concentrations were added to microplate with magnetic inserts in order to study the kinetics of the binding of the particles. The optical absorbance of the particles was measured through the aperture in the centre of the insert. Among the tested nanoparticles were ferrofluid (particles diameter 90 nm), ferromagnetic particles Turbobeads (diameter- 258 nm) and two different paramagnetic MIPs with particles diameter 260 nm and 442 nm prepared in Cranfield (as described below).
The optical absorption of the suspensions of the magnetic particles in microplate with magnetic inserts was measured using microtiter plate reader (Dynex, UK) at 600 nm. Magnetic particles of all sizes tested in this experiment were adsorbed by inserts in 2-3 minutes >90% of the (Figure 3).

**Example 3** - **Assay for melamine using imprinted nanoparticles containing iron oxide as synthetic receptors and blue nanoparticles as reporter elements.**

*3.1. Synthesis of magnetic molecularly imprinted polymer nanoparticles imprinted with melamine.*

Glass beads (75 $\mu$m diameter) were boiled in 4 M NaOH for 10 min, washed with double-distilled water followed by acetone and dried at 80 °C. Beads were incubated in a 2% v/v solution of 3-aminopropyltrimethyloxysilane in toluene overnight, washed with acetone, incubated with a 5% v/v solution of glutare aldehyde in PBS buffer pH 7.2 for 2 h, and rinsed with double-distilled water.
Activated beads were incubated in solution of melamine (5 mg/ml) in PBS pH 7.2 (with 10% v/v pirrolidone) overnight at 4 °C. Finally, the glass beads were washed with water and dried under vacuum. For melamine imprinted nanoparticles, the polymerisation mixture was prepared by mixing 2.88 g methacrylic acid, 3.24 g trimethylolpropane trimethacrylate and 3.24 g ethylene glycol dimethacrylate, 0.78 g N,N-diethyldithiocarbamic acid benzyl ester and 0.18 g pentaerythritol-*tetrakis*-(3-mercaptopropionate) in 10.5 g ACN (total monomer concentration: 70% w/v). 0.7 ml of a dispersion of $Fe_2O_3$ with average particle size of 14 nm was added to this mixture.
The mixture was bubbled with $N_2$ for 10 min and added to 60 g of glass beads with immobilised melamine in a shallow glass beaker. The mixture was placed under UV irradiation for 2.5 minutes. After polymerisation, the beads were placed in a polyethylene SPE cartridge fitted with a 20 micron frit at the bottom and washed with 8 bed volumes of acetonitrile at 4 °C in order to remove unreacted monomers and low affinity polymer. High affinity particles were removed by increasing the temperature of the cartridge and beads to 60 °C and washing with 4 bed volumes of acetonitrile at 60 °C. The size of obtained particles, as determined by dynamic light scattering was ca. 380 nm.

*3.2 Colorimetric assay for melamine.*

150 $\mu$l aliquots of aqueous suspension of magnetic MIP nanoparticles with diameter 442 nm (1 mg/ml) were added to microplate well with magnetic inserts. As a control the wells with magnetic inserts without MIP nanoparticles were also prepared. The optical absorbance of the particles was measured through the aperture in the centre of the insert at wavelength 600 nm using Dynex microtiter plate reader.
The magnetic MIPs were incubated for 5 min until the majority of them was immobilised onto the magnetic inserts. The success of immobilisation manifested as a reduction of the optical density of the suspension in the centre of the well. The kinetics of immobilisation was monitored using microplate reader. 30 $\mu$l of the blue amino-coated Estapore nanoparticles with concentration 0.25 mg/ml (258 nm diameter) modified with melamine were added to each well. The kinetics of the binding of the melamine-coated Estapore particles onto the magnetic inserts with immobilised magnetic MTPs specific for melamine was monitored for 1 hour.
The optical absorbance values were recorded at 600 nm using Dynex mictoplate reader. In order to perform the displacement of the blue Estapore particles from the magnetic inserts with immobilised melamine-specific magnetic MIPs 50 $\mu$l of 100 $\mu$M melamine in 20 mM phosphate buffer pH 7.4 were added and solution was mixed. The displacement effect was measured after the incubation for 5 min. The results are provided in Table 1 which shows optical absorbance of the coloured nanoparticles with immobilised melamine in microplate with magnetic inserts with and without added magnetic MIP nanoparticles imprinted with melamine and in the presence of 100 $\mu$M melamine.

**Table 1**

| Free melamine, $\mu$M | Blue Estapore nanoparticles with immobilised melamine | |
|---|---|---|
| | Without magnetic MIP nanoparticles | With magnetic MIP nanoparticles |
| 0 | 0.3±0.03 | 0.15±0.01 |
| 100 | 0.35±0.03 | 0.27±0.02 |
| Displacement, % | 16% | 80% |

The calculation of the percentage of displacement was done accordingly to equation:

$$x = (OD_m - OD_o) \times 100 / OD_o,$$

where $OD_o$ is the optical density before and $OD_m$- after the addition of melamine.

**Claims**

1. A microplate having a plurality of wells, the plate comprising one or more inserts which are magnets for attracting magnetisable nano- and microparticles, and wherein each of said inserts either:

   fits removably into one of the wells; or
   is integral with or attached to the bottom or side wall inside the well,

   the insert having a hole therethrough along the axis of the well.

2. A microplate according to claim 1, wherein the insert is an annular insert.

3. A microplate according to claim 1 or claim 2, wherein the insert comprises a polymer, glass or ceramic material and a magnetic material.

4. A microplate according to claim 3, wherein the insert comprises:

   a polymer material selected from divinylbenzene, divinylnaphthalene, vinylpyridine, hydroxyalkylene methacrylates, ethylene glycol dimethacrylate, vinyl esters of carboxylic acids, divinyl ether, pentaerythritol di-, tri-, or tetra- methacrylate or acrylate, trimethylopropane trimethacrylate or triacrylate, alkylene bis acrylamides or methacrylamides, methacrylic and acrylic acid, acrylamide, hydroxyethyl methacrylate, epoxy and urethane resins, molecularly imprinted polymers, chitosan, carbohydrates, oligo- and polysaccharides, peptides, proteins and nucleic acid derivatives, agarose, lipids; or
   a material selected from silica gel, glass, acrylamide gel, and polysaccharides.

5. A microplate according to any one of the preceding claims, wherein the radial thickness of the wall of the insert is 1 to 5 mm.

6. A microplate according to any one of the preceding claims, wherein the insert is attached to the well surface by physical or chemical attachment selected from thermal-welding, sonic-welding, infrared-welding, solvent-welding, and use of an adhesive.

7. A microplate according to any one of the preceding claims, wherein a surface of the insert comprises a photoreactive group or comprises a functional group that is capable of covalent attachment of a ligand molecule by chemical reaction to form a Schiff base, disulfide bond, peptide bond, S-metal bond, or ester group, or by chemical reaction with an acetal or thioacetal group on the ligand.

8. A microplate according to any one of the preceding claims, wherein the plate comprises a plurality of the magnetic inserts in the same well.

9. A microplate according to claim 8, wherein the plurality of magnetic inserts comprise at least two different types of insert.

10. A microplate according to claim 9, wherein the at different types of insert differ in one or more of: construction material, magnetic properties, or axial thickness.

11. A magnetic insert as disclosed in claim 1, for use in a microplate according to any one of the preceding claims.

12. Use of a microplate according to any one of the preceding claims:

> in the capture or immobilisation of magnetic particles;
> in the capture or immobilisation of magnetic particles carrying chemical or biological ligands;
> in solid phase synthesis, combinatorial chemistry or high-throughput synthesis;
> in diagnostic testing, enzyme immunoassay, clinical or environmental assay;
> in high throughput screening for genomics, proteomics, or point-of-care in vitro diagnostics;
> in molecular genetic analysis and nucleic acid diagnostics;
> in high throughput screening of materials for separation and catalysis; or
> as a cell culture support in high-throughput screening (HTS) for the discovery or development of new therapeutic drugs.

**Patentansprüche**

1. Mikroplatte, die eine Vielzahl von Wells aufweist, wobei die Platte ein oder mehrere Einsätze umfasst, die Magneten zum Anziehen von magnetisierbaren Nano- und Mikroteilchen sind, wobei jeder der Einsätze entweder:

> entfernbar in einen der Wells passt; oder
> mit dem Boden oder der Seitenwand innerhalb des Wells einstückig ausgebildet oder daran angebracht ist, wobei der Einsatz ein Loch entlang der Achse des Wells aufweist.

2. Mikroplatte nach Anspruch 1, wobei der Einsatz ein ringförmiger Einsatz ist.

3. Mikroplatte nach Anspruch 1 oder 2, wobei der Einsatz ein Polymer-, Glas- oder Keramikmaterial und ein magnetisches Material umfasst.

4. Mikroplatte nach Anspruch 3, wobei der Einsatz Folgendes umfasst:

> ein Polymermaterial, das aus Divinylbenzol, Divinylnaphthalin, Vinylpyridin, Hydroxyalkylenmethacrylaten, Ethylenglykoldimethacrylat, Vinylestern von Carbonsäuren, Divinylether, Pentaerythritdi-, -tri- oder -tetramethacrylat oder -acrylat, Trimethylpropantrimethacrylat oder -triacrylat, Alkylenbisacrylamiden oder -methacrylamiden, Methacryl- und Acrylsäure, Acrylamid, Hydroxyethylmethacrylat, Epoxy- und Urethanharzen, molekular geprägten Polymeren, Chitosan, Kohlenhydraten, Oligo- und Polysacchariden, Peptiden, Proteinen und Nucleinsäurederivaten, Agarose, Lipiden ausgewählt ist; oder
> ein Material, das aus Silicagel, Glas, Acrylamidgel und Polysacchariden ausgewählt ist.

5. Mikroplatte nach einem der vorangegangenen Ansprüche, wobei die radiale Dicke der Wand des Einsatzes 1 bis 5 mm beträgt.

6. Mikroplatte nach einem der vorangegangenen Ansprüche, wobei der Einsatz an der Welloberfläche durch physikalisches oder chemisches Anbringen, das aus Wärmeschweißen, Schallschweißen, Infrarotschweißen, Lösungsmittelschweißen und Verwendung eines Haftmittels ausgewählt ist, angebracht ist.

7. Mikroplatte nach einem der vorangegangenen Ansprüche, wobei eine Oberfläche des Einsatzes eine photoreaktive Gruppe umfasst oder eine funktionelle Gruppe umfasst, die zu kovalenter Bindung eines Ligandenmoleküls durch chemische Reaktion, um eine Schiffsche Base, Disulfidbindung, Peptidbindung, S-Metallbindung oder Estergruppe zu bilden, oder durch chemische Reaktion mit einer Acetal- oder Thioacetalgruppe auf dem Liganden in der Lage ist.

8. Mikroplatte nach einem der vorangegangenen Ansprüche, wobei die Platte eine Vielzahl der magnetischen Einsätze in demselben Well umfasst.

9. Mikroplatte nach Anspruch 8, wobei die Vielzahl von magnetischen Einsätzen zumindest zwei verschiedene Einsatztypen umfasst.

10. Mikroplatte nach Anspruch 9, wobei sich die verschiedenen Einsatztypen in Bezug auf eines oder mehrere aus Konstruktionsmaterial, magnetischen Eigenschaften oder axialer Dicke unterscheiden.

11. Magnetischer Einsatz nach Anspruch 1 zur Verwendung in einer Mikroplatte nach einem der vorangegangenen Ansprüche.

12. Verwendung einer Mikroplatte nach einem der vorangegangenen Ansprüche:

beim Einfangen oder Immobilisieren magnetischer Teilchen;
beim Einfangen oder Immobilisieren magnetischer Teilchen, die chemische oder biologische Liganden tragen;
bei der Festphasensynthese, kombinatorischen Chemie oder Hochdurchsatzsynthese;
bei diagnostischen Tests, Enzymimmuntests, klinischen oder Umwelttests;
beim Hochdurchsatzscreening für Genomik, Protemoik oder Vor-Ort-in-vitro-Diagnostik;
bei der Molekulargenetikanalyse und Nucleinsäurediagnostik;
beim Hochdurchsatzscreening von Materialien zur Trennung und Katalyse; oder
als Zellkulturträger beim Hochdurchsatzscreening (HTS) für die Entdeckung oder Entwicklung neuer therapeutischer Arzneimittel.

## Revendications

1. Microplaque ayant une pluralité de puits, la plaque comprenant un ou plusieurs inserts qui sont des aimants pour attirer des nano- et micro-particules magnétisables, et dans laquelle chacun desdits inserts :

soit s'ajuste de façon amovible dans l'un des puits ;
soit est solidaire avec le fond ou la paroi latérale à l'intérieur du puits ou est attaché à celui-ci,
l'insert ayant un trou traversant le long de l'axe du puits.

2. Microplaque selon la revendication 1, dans laquelle l'insert est un insert annulaire.

3. Microplaque selon la revendication 1 ou la revendication 2, dans laquelle l'insert comprend un matériau en polymère, verre ou céramique, et un matériau magnétique.

4. Microplaque selon la revendication 3, dans laquelle l'insert comprend :

un matériau polymère choisi parmi le divinylbenzène, le divinylnaphtalène, la vinylpyridine, les méthacrylates d'hydroxyalkylène, le diméthacrylate d'éthylèneglycol, les esters vinyliques d'acides carboxyliques, le divinyléther, le di-, tri- ou tétra-méthacrylate ou acrylate de pentaérythritol, le triméthacrylate ou triacrylate de triméthylolpropane, les alkylènebis-acrylamides ou méthacrylamides, l'acide méthacrylique ou acrylique, l'acrylamide, le méthacrylate d'hydroxyéthyle, les résines époxy et d'uréthane, les polymères à impression moléculaire, le chitosane, les hydrates de carbone, les oligo- et poly-saccharides, les peptides, les protéines et les dérivés d'acides nucléiques, l'agarose, les lipides ; ou
un matériau choisi parmi le gel de silice, le verre, le gel d'acrylamide, et les polysaccharides.

5. Microplaque selon l'une quelconque des revendications précédentes, dans laquelle l'épaisseur radiale de la paroi de l'insert est de 1 à 5 mm.

6. Microplaque selon l'une quelconque des revendications précédentes, dans laquelle l'insert est attaché à la surface du puits par un attachement physique ou chimique choisi parmi un soudage thermique, un soudage sonique, un soudage aux infrarouges, un soudage au solvant, et l'utilisation d'un adhésif.

**7.** Microplaque selon l'une quelconque des revendications précédentes, dans laquelle une surface de l'insert comprend un groupe photoréactif ou comprend un groupe fonctionnel qui est capable d'un attachement covalent d'une molécule de ligand par réaction chimique pour former une base de Schiff, une liaison disulfure, une liaison peptide, une liaison S-métal, ou un groupe ester, ou par réaction chimique avec un groupe acétal ou thioacétal sur le ligand.

**8.** Microplaque selon l'une quelconque des revendications précédentes, dans laquelle la plaque comprend une pluralité d'inserts magnétiques dans le même puits.

**9.** Microplaque selon la revendication 8, dans laquelle la pluralité d'inserts magnétiques comprend au moins deux types différents d'inserts.

**10.** Microplaque selon la revendication 9, dans laquelle les différents types d'inserts diffèrent par un ou plusieurs parmi : le matériau de construction, les propriétés magnétiques, et l'épaisseur axiale.

**11.** Insert magnétique tel que divulgué dans la revendication 1, pour une utilisation dans une microplaque selon l'une quelconque des revendications précédentes.

**12.** Utilisation d'une microplaque selon l'une quelconque des revendications précédentes :

  dans la capture ou l'immobilisation de particules magnétiques ;
  dans la capture ou l'immobilisation de particules magnétiques portant des ligands chimiques ou biologiques ;
  dans la synthèse en phase solide, la chimie combinatoire ou la synthèse à haut débit ;
  dans le test diagnostic, l'immunodosage enzymatique, le dosage clinique ou environnemental ;
  dans le criblage à haut débit d'éléments génomiques, protéomiques, ou les diagnostics in vitro sur le lieu d'intervention ;
  dans l'analyse génétique moléculaire et les diagnostics d'acides nucléiques ;
  dans le criblage à haut débit de matériaux pour la séparation et la catalyse ; et
  en tant que support de culture cellulaire dans un criblage à haut débit (HTS) pour la découverte ou le développement de nouveaux médicaments thérapeutiques.

a)

b) individual inserts

c) stacks of inserts

stack of inserts of
similar thickness

stack of inserts of
dissimilar thickness

side elevation view

Fig. 1

Fig. 2

Fig. 3

Fig. 4

**EP 2 856 162 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7449307 B
- US 7332328 B
- US 7219800 B
- US 7384779 B
- US 6040171 A
- US 4230685 A
- US 4554088 A
- US 4628037 A
- US 5779907 A
- US 6954128 B2
- US 7632405 B
- US 20020098121 A
- WO 2009152092 A